(19)

Europäisches Patentamt
European Patent Office
Office européen des brevets

(11) **EP 4 595 884 B1**

(12) **EUROPEAN PATENT SPECIFICATION**

(45) Date of publication and mention
of the grant of the patent:
**11.02.2026 Bulletin 2026/07**

(21) Application number: **25154580.2**

(22) Date of filing: **29.01.2025**

(51) International Patent Classification (IPC):
**A61B 5/389** (2021.01)    **A61B 5/00** (2006.01)

(52) Cooperative Patent Classification (CPC):
**A61B 5/389; A61B 5/7203**

(54) **METHOD AND SYSTEM FOR SIGNAL ELEVATION BASED MUSCLE ACTIVITY DETECTION**

VERFAHREN UND SYSTEM ZUR ERKENNUNG VON MUSKELAKTIVITÄTEN AUF DER BASIS
VON SIGNALERHEBUNGEN

PROCÉDÉ ET SYSTÈME DE DÉTECTION D'ACTIVITÉ MUSCULAIRE BASÉE SUR L'ÉLÉVATION
DE SIGNAL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **01.02.2024 IN 202421006891**

(43) Date of publication of application:
**06.08.2025 Bulletin 2025/32**

(73) Proprietor: **Tata Consultancy Services Limited
Maharashtra (IN)**

(72) Inventors:
• **JAISWAL, Dibyanshu
700135 Kolkata, West Bengal (IN)**
• **PATRO, Prashant Raj
560066 Bangalore, Karnataka (IN)**
• **RAMAKRISHNAN, Ramesh Kumar
560066 Bangalore, Karnataka (IN)**
• **GHOSH, Shubhrangshu
700135 Kolkata, West Bengal (IN)**
• **PAL, Arpan
700135 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(56) References cited:
• YE Y ET AL: "Empirical mode decomposition: a method to reduce low frequency interferences from surface electroenterogram", MEDICAL & BIOLOGICAL ENGINEERING & COMPUTING, SPRINGER, BERLIN, DE, vol. 45, no. 6, 30 May 2007 (2007-05-30), pages 541 - 551, XP019835094, ISSN: 1741-0444
• QIANG YANG ET AL: "ArmIn", MOBILE AND UBIQUITOUS SYSTEMS: COMPUTING, NETWORKING AND SERVICES, ACM, 2 PENN PLAZA, SUITE 701NEW YORKNY10121-0701USA, 5 November 2018 (2018-11-05), pages 117 - 126, XP058423130, ISBN: 978-1-4503-6093-7, DOI: 10.1145/3286978.3287030

EP 4 595 884 B1

## Description

CROSS-REFERENCE TO RELATED APPLICATIONS AND PRIORITY

The present application claims priority to Indian application no. 202421006891, filed on February 01, 2024.

TECHNICAL FIELD

The disclosure herein generally relates to signal processing, and, more particularly, to signal elevation based muscle activity detection in signal processing.

BACKGROUND

Surface Electromyography (sEMG) has emerged as a pivotal tool in the arena of biomechanics, rehabilitation, and movement science. This diagnostic procedure measures the electrical activity produced by skeletal muscles, providing insights into muscle function and signaling pathways. The resulting sEMG signals encapsulate the neuromuscular activities and are often used to analyze muscle response, detect muscle related medical abnormalities, and assist in robotic control. Central to the effective utilization of sEMG signals is the ability to pinpoint the exact moments when the muscle activates (onset) and ceases to be active (offset). Onset and offset detection plays a major role in numerous applications: a) Clinical Analysis: Precise onset and offset determination aids clinicians in diagnosing neuromuscular diseases, determining the extent of muscle injuries, and tracking rehabilitation progress. b) Prosthetics and Orthotics: In the realm of human-computer interaction, particularly in controlling prosthetic limbs, the latency between a user's intent and the device's action must be minimal. Accurate detection of muscle activity onset ensures this promptness. c) Robotics and Human-Robot Collaboration: In environments where humans and robots collaboratively operate, such as industrial assembly lines or therapeutic settings, the synchronization of human muscle activity with robotic motion is a vast field. Accurate onset/offset detection in real-time ensures that robots can anticipate and mirror human actions, significantly reducing the risk of mishaps and enhancing overall operational safety. d) Sports Science: Athletes and trainers use EMG to optimize performance, where even milliseconds of delay in muscle activation can affect outcomes. Accurate onset detection helps in fine-tuning training regimens. However, the chaotic nature of raw EMG signals, contaminated with noise and interference from various sources, often complicates the task of accurate onset/offset detection. For the same reason, existing signal processing systems struggle to perform the onset and offset detection effectively, which in turn affects end applications. Accurate detection not only enhances the quality of the data derived from EMG but also amplifies its applicability across diverse domains, from healthcare to assistive robotics.

A system for surface EMG processing based on empirical mode decomposition is known from YE Y ET AL: "Empirical mode decomposition: a method to reduce low frequency interferences from surface electroenterogram", MEDICAL & BIOLOGICAL ENGINEERING & COMPUTING, vol. 45, no. 6, pages 541-551.

SUMMARY

Embodiments of the present disclosure present technological improvements as solutions to one or more of the above-mentioned technical problems recognized by the inventors in conventional systems. In accordance with the invention, a processor implemented method is provided. The method includes: receiving, via one or more hardware processors, a plurality of raw sEMG signals (sEMGr) of a subject, as input; preprocessing, via the one or more hardware processors, the plurality of sEMGr signals obtain an sEMG envelope; performing, via the one or more hardware processors, signal elevation on the sEMG envelope to obtain a conditioned signal, by: decomposing the sEMG envelope into a plurality of Intrinsic Mode Functions (IMF); determining IMF having a) least value of noise, and b) a total power range with closest match with the sEMGr, from among the plurality of IMFs, as candidate IMF; and elevating the sEMG envelope by multiplying the sEMG envelope with the determined candidate IMF, to generate a plurality of sEMGe signals for a plurality of channels; determining, via the one or more hardware processors, onset and offset regions in the sEMGe signal from each of the plurality of channels, by performing segmentation of the sEMGe signal from each of the plurality of channels; and post-processing, via the one or more hardware processors, the sEMGe signals from the plurality of channels, to generate a combined sEMGe signal, wherein the combined sEMGe signal represents a muscle potential activity of the subject.

In an embodiment of the method, preprocessing the plurality of sEMGr signals to obtain the sEMG envelope comprises of performing a DC offset removal, removal of any powerline noise and associated harmonics, band-pass filtering, full wave rectification, and low-pass filtering, of the sEMGr signals.

In an embodiment of the method, determining the onset and offset regions in the sEMGe signal from each of the plurality of channels, by performing the segmentation, includes: obtaining square waves associated with each of the plurality of channels, by applying an adaptive threshold-based segmentation algorithm on the sEMGe signal from each of the plurality of channels; identifying time instances having a rising edge of the square wave as the onset of an active period; and identifying time instances having a falling edge of the square wave as the offset of the active period.

In an embodiment of the method, generating the combined sEMGe signal comprises of performing a logical **OR** operation of the square waves obtained for the plurality of channels.

In an embodiment of the method, the sEMG envelope is decomposed using a Variational Mode Decomposition (VMD) technique.

Further according to the invention, a system is provided. The system includes one or more hardware processors, a communication interface, and a memory storing a plurality of instructions. The plurality of instructions cause the one or more hardware processors to: receive a plurality of raw sEMG signals (sEMGr) of a subject, as input; preprocess the plurality of sEMGr signals obtain an sEMG envelope; perform signal elevation on the sEMG envelope to obtain a conditioned signal, by: decomposing the sEMG envelope into a plurality of Intrinsic Mode Functions (IMF); determining IMF having a) least value of noise, and b) a total power range with closest match with the sEMGr, from among the plurality of IMFs, as candidate IMF; and elevating the sEMG envelope by multiplying the sEMG envelope with the determined candidate IMF, to generate a plurality of sEMGe signals for a plurality of channels; determine onset and offset regions in the sEMGe signal from each of the plurality of channels, by performing segmentation of the sEMGe signal from each of the plurality of channels; and post-process the sEMGe signals from the plurality of channels, to generate a combined sEMGe signal, wherein the combined sEMGe signal represents a muscle potential activity of the subject.

In an embodiment of the system, the one or more hardware processors are configured to preprocess the plurality of sEMGr signals to obtain the sEMG envelope by performing a DC offset removal, removal of any powerline noise and associated harmonics, band-pass filtering, full wave rectification, and low-pass filtering, of the sEMGr signals.

In an embodiment of the system, the one or more hardware processors are configured to determine the onset and offset regions in the sEMGe signal from each of the plurality of channels, by performing the segmentation, includes: obtaining square waves associated with each of the plurality of channels, by applying an adaptive threshold-based segmentation algorithm on the sEMGe signal from each of the plurality of channels; identifying time instances having a rising edge of the square wave as the onset of an active period; and identifying time instances having a falling edge of the square wave as the offset of the active period.

In an embodiment of the system, the one or more hardware processors are configured to generate the combined sEMGe signal by performing a logical OR operation of the square waves obtained for the plurality of channels.

**In** an embodiment of the system, the one or more hardware processors are configured to decompose the sEMG envelope using a Variational Mode Decomposition (VMD) technique.

Further according to the invention, a non-transitory computer readable medium is provided. The non-transitory computer readable medium includes a plurality of instructions which causes one or more hardware processors to: receive a plurality of raw sEMG signals (sEMGr) of a subject, as input; preprocess the plurality of sEMGr signals obtain an sEMG envelope; perform signal elevation on the sEMG envelope to obtain a conditioned signal, by: decomposing the sEMG envelope into a plurality of Intrinsic Mode Functions (IMF); determining IMF having a) least value of noise, and b) a total power range with closest match with the sEMGr, from among the plurality of IMFs, as candidate IMF; and elevating the sEMG envelope by multiplying the sEMG envelope with the determined candidate IMF, to generate a plurality of sEMGe signals for a plurality of channels; determine onset and offset regions in the sEMGe signal from each of the plurality of channels, by performing segmentation of the sEMGe signal from each of the plurality of channels; and post-process the sEMGe signals from the plurality of channels, to generate a combined sEMGe signal, wherein the combined sEMGe signal represents a muscle potential activity of the subject.

In an embodiment of the non-transitory computer readable medium, preprocessing the plurality of sEMGr signals to obtain the sEMG envelope comprises of performing a DC offset removal, removal of any powerline noise and associated harmonics, band-pass filtering, full wave rectification, and low-pass filtering, of the sEMGr signals.

In an embodiment of the non-transitory computer readable medium, determining the onset and offset regions in the sEMGe signal from each of the plurality of channels, by performing the segmentation, includes: obtaining square waves associated with each of the plurality of channels, by applying an adaptive threshold-based segmentation algorithm on the sEMGe signal from each of the plurality of channels; identifying time instances having a rising edge of the square wave as the onset of an active period; and identifying time instances having a falling edge of the square wave as the offset of the active period.

In an embodiment of the non-transitory computer readable medium, generating the combined sEMGe signal comprises of performing a logical OR operation of the square waves obtained for the plurality of channels.

In an embodiment of the non-transitory computer readable medium, the sEMG envelope is decomposed using a Variational Mode Decomposition (VMD) technique.

In an embodiment of the non-transitory computer readable medium, the one or more hardware processors are configured to decompose the sEMG envelope using a Variational Mode Decomposition (VMD) technique.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A illustrates an exemplary system for signal elevation based muscle activity detection, according to some embodiments of the present disclosure.

FIG. 1B illustrates an example implementation of the system of FIG. 1, according to some embodiments of the present disclosure.

FIGS. 2A and 2B (collectively referred to as FIG. 2) is a flow diagram depicting steps involved in the process of signal elevation based muscle activity detection by the system of FIG. 1, according to some embodiments of the present disclosure.

FIG. 3 is a flow diagram depicting steps involved in the process of determining the onset and offset regions in surface Electromyography (sEMGe) signal from each of a plurality of channels, in accordance with some embodiments of the present disclosure.

FIGS. 4A through 4C are graphs depicting example values associated with different stages of the signal elevation based muscle activity detection, as being performed by the system of FIG. 1, in accordance with some embodiments of the present disclosure.

FIG. 4D illustrates balanced accuracy metrics for Onset, in the signal elevation based muscle activity detection being performed by the system of FIG. 1, in accordance with some embodiments of the present disclosure.

FIGS. 5A and 5B illustrate cluster formation for a good channel and a bad channel respectively, in the signal elevation based muscle activity detection being performed by the system of FIG. 1, in accordance with some embodiments of the present disclosure.

FIG. 6 illustrates separation of purity index for the good and bad channels respectively, in the signal elevation based muscle activity detection being performed by the system of FIG. 1, in accordance with some embodiments of the present disclosure.

FIG. 7 illustrates comparison of different methods in an experimental set up of the signal elevation based muscle activity detection being performed by the system of FIG. 1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Accurate onset detection helps in fine-tuning training regimens. However, the chaotic nature of raw EMG signals, contaminated with noise and interference from various sources, often complicates the task of accurate onset/offset detection. For the same reason, existing signal processing systems struggle to perform the onset and offset detection effectively, which in turn affects end applications. Accurate detection not only enhances the quality of the data derived from EMG but also amplifies its applicability across diverse domains, from healthcare to assistive robotics.

To address these challenges, a signal elevation based muscle activity detection approach is provided. In this method, a plurality of raw Surface Electromyography (sEMG) signals (sEMGr) of a subject are received, via one or more hardware processors, as input. Further, the plurality of sEMGr signals are preprocessed, via the one or more hardware processors, to obtain an sEMG envelope. Further, signal elevation is performed on the sEMG envelope, via the one or more hardware processors, to obtain a conditioned signal, by: a) decomposing the sEMG envelope into a plurality of Intrinsic Mode Functions (IMF), b) determining IMF having a) least value of noise, and b) a total power range with closest match with the sEMGr, from among the plurality of IMFs, as candidate IMF, c) and elevating the sEMG envelope by multiplying the sEMG envelope with the determined candidate IMF, to generate a plurality of sEMGe signals for a plurality of channels. Further, onset and offset regions in the sEMGe signal are determined, via the one or more hardware processors, from each of the plurality of channels, by performing segmentation of the sEMGe signal from each of the plurality of channels. Further, the sEMGe signals from the plurality of channels are post-processed, via the one or more hardware processors, to generate a combined sEMGe signal, wherein the combined sEMGe signal represents a muscle potential activity of the subject. In this approach, achieving the signal elevation enables the onset and offset detection, thereby aiding end applications.

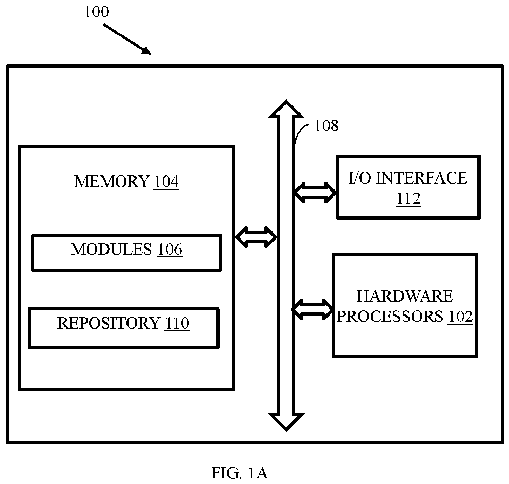

Referring now to the drawings, and more particularly to FIG. 1A through FIG. 7, where similar reference characters denote corresponding features consistently throughout the figures, there are shown preferred embodiments and these embodiments are described in the context of the following exemplary system and/or method.

FIG. 1A illustrates an exemplary system for signal elevation based muscle activity detection, to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of signal elevation based onsite and offset detection in signal processing. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the signal elevation based onsite and offset detection in signal processing.

The data repository (or repository) 110 may include a plurality of abstracted piece of code for refinement and data that is processed, received, or generated as a result of the execution of the plurality of modules in the module(s) 106.

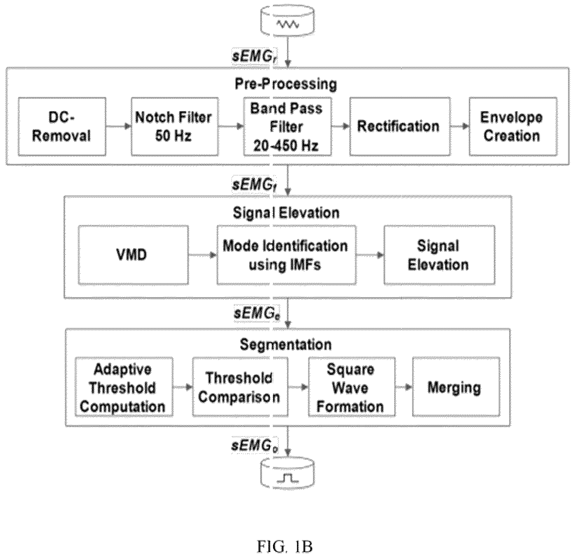

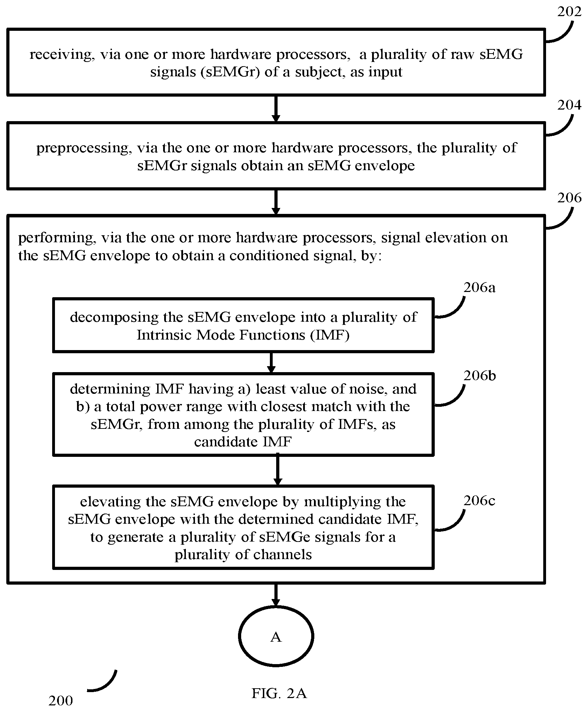

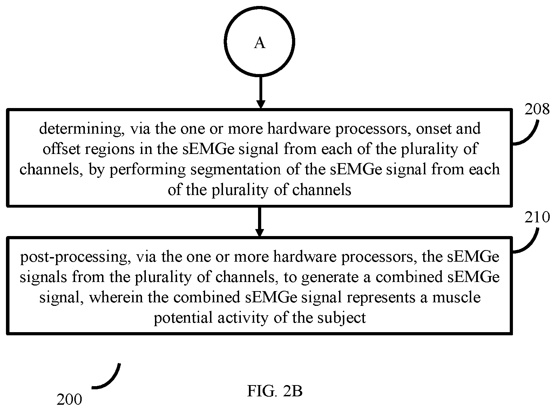

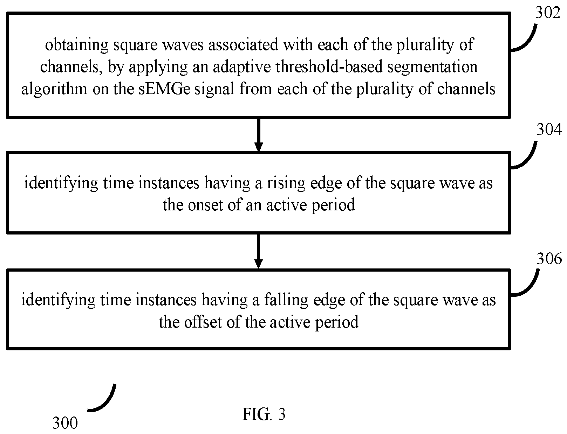

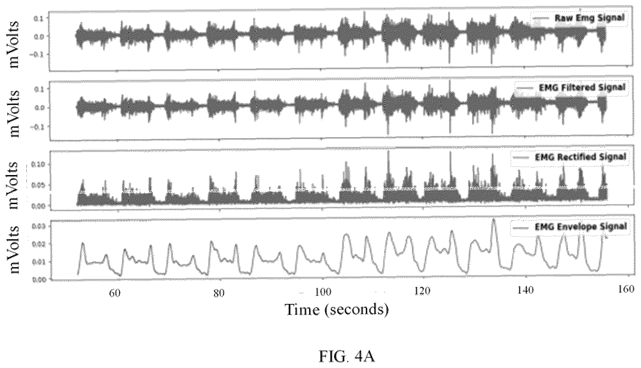

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). The example implementation of the system 100 as depicted in FIG. 1B shows three stages, i.e., pre-processing, signal elevation, and segmentation. The three stages, and functions of the components of the system 100 in FIGS. 1A and 1B are now explained with reference to steps in flow diagrams in FIG. 2 and FIG. 3, and the example graphs depicted in FIGS. 4A through 4C.

FIGS. 2A and 2B (collectively referred to as FIG. 2) is a flow diagram depicting steps of a method 200 involved in the process of signal elevation based muscle activity detection by the system of FIG. 1, according to some embodiments of the present disclosure. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the system 100 receives, via the one or more hardware processors 102, a plurality of raw sEMG signals (sEMGr) of a subject, as input. The subject herein refers to a person whose health is being monitored.

Further, at step 204 of the method 200, the system 100 preprocesses the plurality of sEMGr signals obtain an sEMG envelope, via the one or more hardware processors 102. Amplitude of the sEMG signal may range in milli volt scale, with its power spectrum residing predominantly between the 20-450 Hz band. Various applications typically prioritize specific information concerning muscle activity and the degree of muscle contraction. Consequently, the envelope of the EMG signal is often favored over the raw signal for this purpose. To obtain the EMG envelope from the raw signals the following steps are performed. Initially, channel-wise raw sEMG data undergoes Direct Current (DC) offset removal as the current interest lies in the variation in the instantaneous muscle potentials observed across time to mark the onset and offset of the gestures. Further, the signals are passed through a notch filter of 50Hz to remove any powerline noise and its harmonics, followed by a band-pass filter of 20 to 450 Hz. Since, sEMG signals have both positive and negative components, a full wave rectifications is performed, which helps in conserving the energy of the signal. The rectified output is then passed through a 4th order, lowpass Butterworth filter, with a cut-off of 10Hz to obtain the linear envelope of the sEMG signal. The sEMG envelope gives a measure of local signal power, which is used in EMG segmentation indicating the presence or absence of any muscle potential activity and is considered as an output of the pre-processing stage. Outputs of different stages are shown in FIG. 4A.

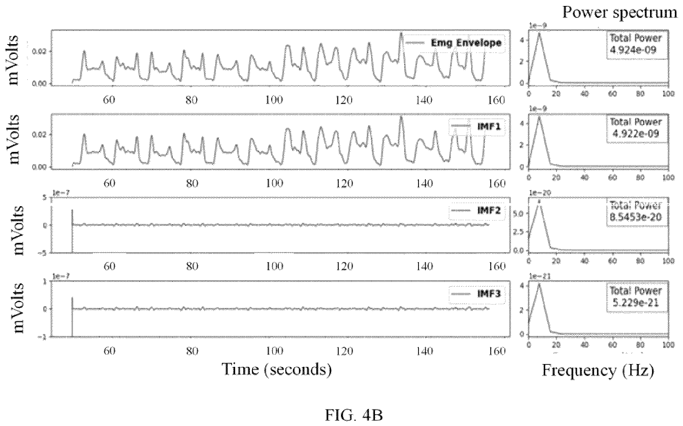

Further, at step 206 of the method 200, the system 100 performs, via the one or more hardware processors 102, signal elevation on the sEMG envelope to obtain a conditioned signal. Various steps involved in the process of performing the signal elevation are depicted in steps 206a through 206c, and are explained hereafter. At step 206a, the system 100 decomposes the sEMG envelope into a plurality of Intrinsic Mode Functions (IMF), example is given in FIG. 4B. The system 100 may use any suitable approach for the purpose of decomposing the signal to the constituent components, i.e. the IMFs. For example, the system 100 may use a Variational Mode Decomposition (VMD) technique for the purpose of decomposing the sEMG envelope. In the context of sEMG signals, the application of VMD allows for the extraction of the intrinsic oscillatory modes, isolating frequency components associated with muscle contractions. In the method 200, the VMD maybe used for obtaining a dominant mode with respect to maximum power of a spectrum obtained for each mode. At this stage, the system 100 may decompose the envelope of the sEMG signal into a pre-determined number of IMFs (referred to as the plurality of IMFs). FIG. 4B depicts plots for the sEMG envelope (first row) and associated IMFs (subsequent rows). Left column gives a time series signal, while on the right the power spectrum of the associated time series is given. Further, at step 206b of the method 200, the system 100 determines an IMF, from among the generated pre-defined number of IMFs, having a) least value of noise, and b) a total power range with closest match with the sEMGr, from among the plurality of IMFs, as candidate IMF. In the frequency domain analysis of the respective spectrum, it is observed that only a first IMF from among the plurality of IDFs has the total power range (1e-9) that matches with the input signal, which is an indication that the first IMF includes a major component of the input signal, and the remaining IMFs of the plurality of IMFs are of very low power (total power in the range of 1e-20 ). In this scenario, the first IMF is determined as the candidate IMF. Further, at step 206c of the method 200, the system 100 elevates the sEMG envelope by multiplying the sEMG envelope with the determined candidate IMF, generating a plurality of sEMGe signals for a plurality of channels.

Further, in segmentation stage, at step 208 of the method 200, the system 100 determines, via the one or more hardware processors 102, onset and offset regions in the sEMGe signal from each of the plurality of channels, by performing segmentation of the sEMGe signal from each of the plurality of channels. Various steps involved in the process of segmentation are depicted in method 300 in FIG. 3, and are explained hereafter. At step 302 of the method 300, the system 100 obtains square waves associated with each of the plurality of channels, by applying an adaptive threshold-based segmentation algorithm on the sEMGe signal from each of the plurality of channels. The adaptive threshold-based segmentation is represented as:

$$\tau(i) = \alpha \mu sEMG_e(i{:}i{+}\omega) + \beta \sigma sEMG_e(i{:}i{+}\omega) \qquad (1a)$$

$$sEMG_o(i) = \begin{cases} 1, \text{if } sEMGe(i) \; > \; \tau(i) \\ 0, \text{otherwise} \end{cases} \qquad (1b)$$

where $\tau$, is computed as a look-ahead adaptive threshold for ith data point, with a window of size $\omega$, and $\alpha$, $\beta$ control the contribution of mean ($\mu$) and standard deviation ($\sigma$) of the window under consideration. $\tau(i)$ is then compared with data point of the sEMG e signal as given in Equation 1b. This is done for all the channels, which are then post processed to obtain a single square wave as the final output.

Each of the square waves has 0's and 1's, where 0's indicate/represent rest regions, and 1's indicate active regions. At step 304 of the method 300, the system 100 identifies time instances having a rising edge of the square wave as the onset of an active period. Similarly, at step 306 of the method 300, the system 100 identifies time instances having a falling edge of the square wave as the offset of the active period.

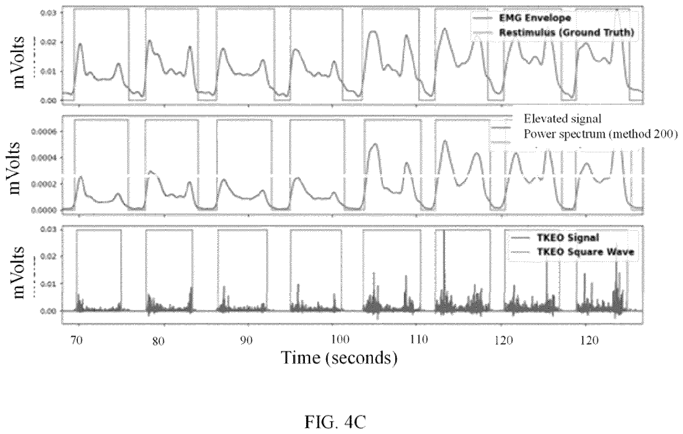

Referring back to method 200, at step 210, the system 100 post-processes, via the one or more hardware processors 102, the sEMGe signals from the plurality of channels, to generate a combined sEMGe signal which represents a muscle potential activity of the subject. The system 100 may generate the combined sEMGe signal by performing a logical OR operation of the square waves obtained for the plurality of channels. Not all muscles are activated for different kinds of gestures and not all muscles are activated for a given gesture. In order to incorporate all the channels, the logical OR operation is performed, thereby generating the combined sEMGe signal. A signal/wave obtained after this post-processing stage is depicted in FIG. 4C, and this combined sEMGe signal is represented as:

$$sEMG_w(t) = sEMG^1_o(t) \vee sEMG^2_o(t) \vee... \vee sEMG^n_o(t) \qquad --- (2)$$

The muscle potential activity thus determined maybe then used for various applications, such as but not limited to the clinical analysis, the prosthetics and orthotics application, and the robotics and human-robot collaboration.

Experimental Data:

Dataset used:-

NinaProDB6 Dataset was used for the experiments conducted. The NinaProDB6 dataset is a component of the NinaPro database, which is a comprehensive collection of data focused on surface electromyographic (sEMG) recordings related to hand movements. The primary goal of the NinaPro database is to support research in the realms of neuro-prosthetics, myoelectric prostheses, and myo-controlled robotics.

The EMG dataset captured various hand and finger movements from multiple subjects using Delsys Trigno Wireless sEMG sensors at a 2000Hz sample rate across 16 channels. Following the NinaPro protocol, it included 7 hand gestures, repeated 12 times daily for 5 days by each of the 10 subjects. Electrodes were placed in two circles around the forearm. This detailed NinaProDB6 dataset facilitated the study of muscle activity transitions, aiding researchers in pinpointing muscle activation and deactivation moments, essential for refining real-time onset and offset detection algorithms.

Validation Approach used:-

1) *Procedure to obtain ground truth from the dataset:* In order to validate the performance of the onset-offset detection approach in method 200, on NinaProDB6, a ground-truth of true onset and offset timestamps of the hand gestures is required. Since the stimulus used was a video stimulus, involving a reaction time by the subject, the stimulus provided rest and gesture timestamps could not be used as ground-truth. This challenge was observed in the very beginning of the NinaPro datasets, and hence an offline *relabelling* was done, that constraints the labels to those regions where there was a high likelihood of increased sEMG activity. The output was termed as *restimulus* and was used as ground truth for this study. For the validation, first 4 channels of sEMG data were used, and the method 200 was applied to perform segmentation and then the output was compared with *restimulus.*

2) *Metrics used for validation:* Initially, Mean Absolute Error (MAE) and Pearson correlation coefficient ($\rho$) were employed as the metrics. These were used to evaluate the alignment of the output generated by the method 200, with the ground truth. A time-step-wise assessment was conducted to derive the MAE and $\rho$ values, taking the full time series of a data session.

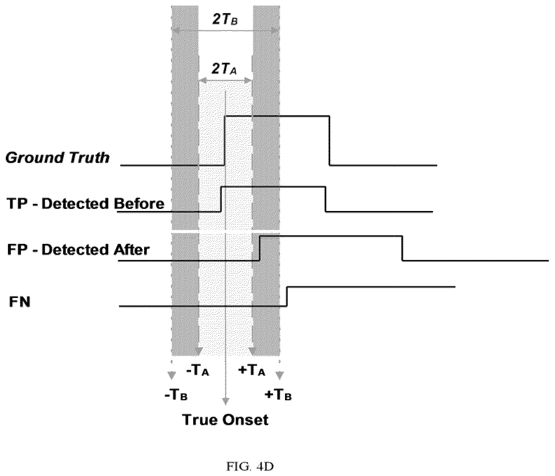

The next primary metric chosen for the validation of segmentation output was Balanced Accuracy (BA). The metric BA gives goodness of a segmentation pipeline of the method 200 in terms of number of segments correctly identified with respect to the ground truth, given tolerable precise proximity. This proximity was defined using a combination of two thresholds ($\pm T_A$ and $\pm T_B$) in terms of duration in milliseconds, w.r.t. the True onset/offset. Balanced Accuracy is computed was the mean of Sensitivity (or True Positive Rate) and Specificity (or True Negative Rate). As shown in FIG. 4D, a True Positive (TP) was assigned when the onset was detected within the designated range of $\pm T_A$, w.r.t the True Onset. Conversely, a False Positive (FP) was marked when an onset was detected but falls in the range of $\pm(T_B - T_A)$. A False Negative (FN) denotes scenarios where no onset is detected or is detected outside range $\pm T_B$. A True Negative (TN) was counted when REST segments were observed without any false positives. A similar interpretation was used for offsets. Furthermore, the time precision was also important for the correct onset and offset detected. In this case the duration (in milliseconds) before or after, of the detected onset/offset ($t_{detected}$) w.r.t the true onset/offset ($t_{true}$) gives the time precision measures of the detected onset/offset. These two metrics gave the magnitude of error. The method 200 was evaluated w.r.t to the average duration across all the instances of onset and offset detected. At first, these were recorded as individual duration of onset/offsets detected before ($d_{bfr}$) the true time instant and duration of onset/offsets detected after the true time instant($d_{aft}$). The number of before and after instances were also recorded as $c_{bfr}$ and $c_{aft}$ respectively. Finally, averaging was done across the all number of instances of before and after onset/offset across the dataset.

$$d = t_{detected} - t_{true}$$

$$d_{\mathrm{bfr}} = d \text{ if } d < 0 \qquad (4) \; d_{\mathrm{aft}} = d \text{ if } d \geq 0$$

*3) Cluster Purity based Channel Rejection:* During our analysis, it was found that for a few subjects, the proposed pipeline nor the TKEO pipeline were performing well. On closer investigation into the subjects brought us to the conclusion that for few subjects, once the *sEMGe* is obtained, all channels did not have good signal quality. Given the protocol of NinaPro Database, the sequence consists of alternating rest and active segments, we were unable to compute the signal-to-noise ratio (SNR) since it requires a proper baseline data in order to model the random noise in the signal. Hence we had to take an alternative route to identify the channels which did not have proper signatures throughout. Though, by visual inspection the bad channels were identifiable, to validate our observation we used cluster purity based channel rejection method.

In this method (as shown in Algorithm *1), restimulus* was treated as the ground truth, to segment the signal into *rest* (label 0) and *active* (label 1) segments. For each such segment, mean and standard deviation was computed and recorded. Once all the segments were processed for the input channel, *kmeans* clustering was performed *(k=2)* using computed mean and standard deviation as features. In an ideal scenario the data points from *rest* segments would have mean and standard deviation values lower than a set threshold. On the contrary, segments from *active* regions would have mean and standard deviation values higher than the threshold value, hence the segments would be grouped into 2 distinct clusters (Cluster 0 and Cluster 1) corresponding to *rest* and *active* labels. The clustered output along with the segment labels was then used to compute the channel purity index as given by Equation 5:

$$\text{Purity} = \frac{1}{n} \sum_{k=0}^{K-1} \frac{max}{j} (\omega_k, j) \qquad --- (5)$$

where: *n* is the total number of segments. *K* is the total number of clusters and $w_{k,j}$ represents the number of instances in cluster *k* that belong to class *j* = [0, 1].

---------------------------------------------------------------

Algorithm 1: Channel Purity Computation

Data: $S \leftarrow$ Signal divided into segments

$L \leftarrow$ Labels (0,1) corresponding to segments Result: Purity value of the

clustering globalArray $\leftarrow$ empty array; foreach *segment in S* do

    mean $\leftarrow$ computeMean(segment);

    stddev $\leftarrow$ computeStdDev(segment);

    label $\leftarrow$ getLabel(segment,*L*);

    append (mean, stddev, label) to globalArray;

end

clusters $\leftarrow$ Kmeans(*globalArray, 2*);

purityVal $\leftarrow$ Purity(*clusters, L*); return purityVal;

---------------------------------------------------------------

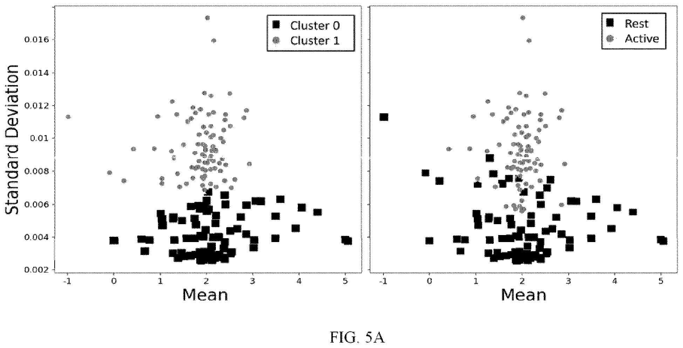

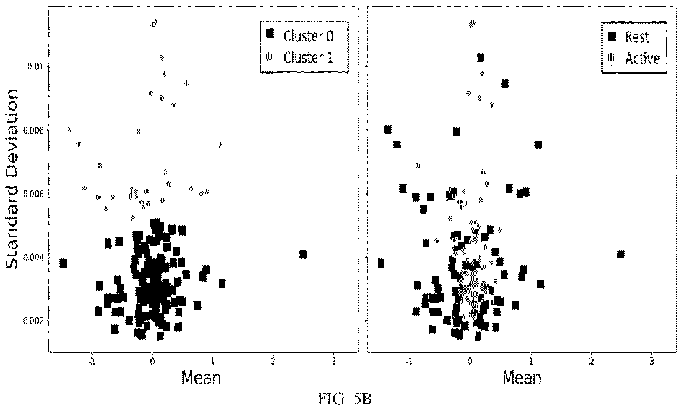

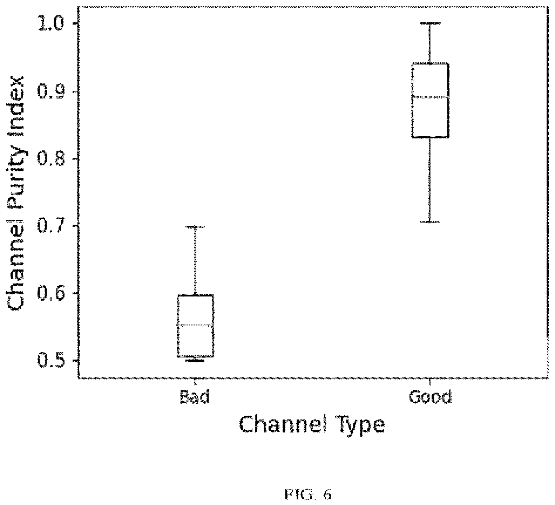

FIG. 5 gives a scatter plot comparison of the segment clusters formed via K-means (left) and ground-truth labels (right). Each point corresponds to a segment and color coded based on the cluster assigned (left) or as per the segment label (right). It was observed that, for the visually inspected good channels, the clustering output (FIG. 5A), assigned cluster indices corresponds well to ground truth labels, indicating high purity value of 0.86 and a good difference in the mean and standard deviation of the *rest* and *active* segments. On the other hand, for the bad channels (FIG. 5B), there assigned cluster indices do not tally well with the ground truth labels, as there is no clear separation w.r.t segment labels also indicated by a low purity value of 0.51 for the channel. Based on visual inspection and categorization of good and bad channels, a box plot of the corresponding purity values is shown in FIG. 6. A clear separation of the purity values was observed for categorized good and bad channels. It is to be noted that the terms 'low', 'high', 'good', and 'bad', are defined with respect to associated threshold.

Results:

For the analysis purpose, results were generated using a *Teager-Kaiser Energy operator Based Signal Conditioning and Segmentation approach.* The Teager-Kaiser energy operator (TKEO) is a nonlinear operator that calculates the energy of mono component signals. TKEO was used to condition surface electromyography (EMG) signals, as it increases the detection accuracy of EMG burst boundaries and highlight motor unit activities. Prior to applying TKEO, the input signal was preprocessed using same steps as being used in the method 200, except that instead of the envelope signal, a filtered signal was used as input to the TKEO. The expression for discrete TKEO is given as:

$$T\left[x(t)\right] = x^2(t) - x\left(t+1\right)x\left(t-1\right)$$

where, x(t) is the given input signal, in this case filtered sEMG signal, and *T* is TKEO conditioned output signal, obtained as function of only 3 instantaneous data points of the input signal, thus providing a high time resolution and good adaptability towards transient changes in the signals. Once a TKEO condition signal was obtained for all the channels, it was then used for segmentation, replacing $\tau(i)$ with $T[x(i)]$. Further post processing was performed. The segmentation output was used for comparison purpose.

Different metrics are tabulated by comparing results obtained using the method 200 with the ground truth and TKEO output with ground truth. Table I reports the *MAE, RMSE* and correlation coefficient ($\rho$) averaged over full dataset. A good association is given by lower MAE value and a high correlation value. The advantage of using *MAE* or *RMSE* is to give the comparison of the error in the same physical quantity i.e. time duration in milli secs. From the table, it is observed that the method 200 fulfills both the criteria and stands better than the TKEO approach.

TABLE I: Segmentation Square Wave Comparison

| TA | TB | Pipeline | MAE | RMSE | $\rho$ |
|---|---|---|---|---|---|
| 0.5 | 0.75 | Proposed | 0.099 | 0.308 | 0.746 |
|  |  | TEAGER | 0.155 | 0.382 | 0.673 |

Table II lists the BA and average $d_{bfr}$ and $d_{aft}$ of the method 200 and that of TKEO, with $T_A = 0.5s$ and $T_B = 0.75s$. The metrics were tabulated separately for onset and offset respectively as edge type of the square wave. First half of the Table II tabulates the results obtained on the full dataset (considering bad channels as well), whereas the lower half of the Table II represents results after applying channels rejection based on visual inspection and channel purity.

Considering onsets only, higher BA was observed for the method 200 as compared to the TKEO approach. It is to be further noted that for the onsets obtained from TKEO approach, the $c_{bfr}$ was very less and $c_{aft}$ was very high as compared to that of the method 200. Which means in a large number of instances TKEO is detecting onsets after the ground truth, indicating delay in detection of the onsets. On the other hand, BA for offsets turn out to better for TKEO than that of the method 200. But $c_{bfr}$ was found to be very high for TKEO as compared to $c_{aft}$, which implied that in most of the cases, TKEO approach detected offsets earlier than ground truth, indicating the end of gesture before the gesture actually ended.

TABLE II: Balanced Accuracy and Precision of Onset, Offsets

| Pipeline[a] | Edge Type | BA | cbfr | Avg. $d_{bfr}$ | caft | Avg. $d_{aft}$ |
|---|---|---|---|---|---|---|
| Method 200 | ONSET | 0.880 | 3473 | -0.113 | 2893 | 0.133 |
|  | OFFSET | 0.736 | 744 | -0.115 | 3515 | 0.252 |
| TEAGER | ONSET | 0.862 | 500 | -0.167 | 5610 | 0.146 |
|  | OFFSET | 0.790 | 3633 | -0.214 | 1407 | 0.229 |
| Method 200 | ONSET | 0.918 | 2572 | -0.102 | 2364 | 0.122 |
|  | OFFSET | 0.776 | 633 | -0.122 | 2782 | 0.245 |
| TEAGER | ONSET | 0.911 | 385 | -0.164 | 4470 | 0.129 |
|  | OFFSET | 0.828 | 2956 | -0.218 | 995 | 0.227 |

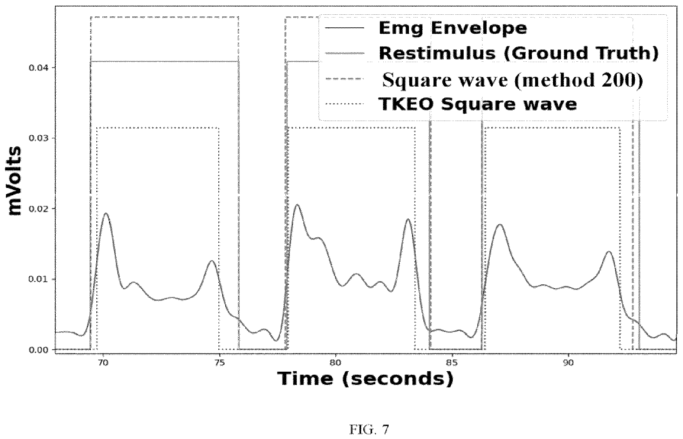

Combining both the inferences, TKEO appeared to be detecting the onsets later and offsets earlier than the ground truth, resulting in detecting shorter segments of active regions. This observation could be clearly seen in a magnified view as in FIG. 7, with all the three square waves i.e. *restimulus* (ground truth), output square wave of method 200, and TKEO method output square wave. Different heights of the square wave were used just for better visualization. It is clear from the plot that the segmentation output of the method 200 shows higher consensus with the ground truth in terms on detection of onset and offsets accurately, as compared to the TKEO method output.

Further, for the lower half of the Table II, which represents better performance metrics as compared to the upper half, is due to the bad channels rejected based on channel purity. On rejecting the bad channels of *sEMG e,* the post-processing output obtained were of better quality. Thus, the improvement in the intermediate stage of obtaining *sEMGo* led to better performance of the final outcome.

The written description describes the subject matter herein to enable any person skilled in the art to make and use the embodiments. The scope of the subject matter embodiments is defined by the claims.

The embodiments of present disclosure herein address unresolved problem of onsite and offset detection in signal processing. The embodiment, thus provides a mechanism for signal elevation detection. Moreover, the embodiments herein further provide a mechanism for signal elevation based onsite and offsite detection in signal processing.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method, comprising:

receiving (202), via one or more hardware processors, a plurality of raw sEMG signals (sEMGr) of a subject, as input;

preprocessing (204), via the one or more hardware processors, the plurality of sEMGr signals to obtain an sEMG envelope;

performing (206), via the one or more hardware processors, signal elevation on the sEMG envelope to obtain a conditioned signal, by:

decomposing (206a) the sEMG envelope into a plurality of Intrinsic Mode Functions (IMF); **characterized in that** the method further comprises:

determining (206b) IMF having a) least value of noise, and b) a total power range with closest match with the sEMGr, from among the plurality of IMFs, as a candidate IMF; and

elevating (206c) the sEMG envelope by multiplying the sEMG envelope with the determined candidate IMF, to generate a plurality of sEMGe signals for a plurality of channels;

determining (208), via the one or more hardware processors, onset and offset regions in the sEMGe signal from each of the plurality of channels, by performing segmentation of the sEMGe signal from each of the plurality of channels; and

post-processing (210), via the one or more hardware processors, the sEMGe signals from the plurality of channels, to generate a combined sEMGe signal, wherein the combined sEMGe signal represents a muscle potential activity of the subject.

2. The processor implemented method as claimed in claim 1, wherein preprocessing the plurality of sEMGr signals to obtain the sEMG envelope comprises of performing a DC offset removal, removal of any powerline noise and associated harmonics, band-pass filtering, full wave rectification, and low-pass filtering, of the sEMGr signals.

3. The processor implemented method as claimed in claim 1, wherein determining the onset and offset regions in the sEMGe signal from each of the plurality of channels, by performing the segmentation, comprises:

obtaining (302) square waves associated with each of the plurality of channels, by applying an adaptive threshold-based segmentation algorithm on the sEMGe signal from each of the plurality of channels;

identifying (304) time instances having a rising edge of the square wave as the onset of an active period; and

identifying (306) time instances having a falling edge of the square wave as the offset of the active period.

4. The processor implemented method as claimed in claim 3, wherein generating the combined sEMGe signal comprises of performing a logical OR operation of the square waves obtained for the plurality of channels.

5. The processor implemented method as claimed in claim 1, wherein the sEMG envelope is decomposed using a Variational Mode Decomposition (VMD) technique.

6. A system (100), comprising:

one or more hardware processors (102);

a communication interface (112); and

a memory (104) storing a plurality of instructions, wherein the plurality of instructions cause the one or more hardware processors to:

receive a plurality of raw sEMG signals (sEMGr) of a subject, as input;

preprocess the plurality of sEMGr signals to obtain an sEMG envelope;

perform signal elevation on the sEMG envelope to obtain a conditioned signal, by:

decomposing the sEMG envelope into a plurality of Intrinsic Mode Functions (IMF); **characterized in that** the plurality of instructions cause the one or more hardware processors to:

determining IMF having a) least value of noise, and b) a total power range with closest match with the sEMGr, from among the plurality of IMFs, as a candidate IMF; and

elevating the sEMG envelope by multiplying the sEMG envelope with the determined candidate IMF, to generate a plurality of sEMGe signals for a plurality of channels;

determine onset and offset regions in the sEMGe signal from each of the plurality of channels, by performing segmentation of the sEMGe signal from each of the plurality of channels; and

post-process the sEMGe signals from the plurality of channels, to generate a combined sEMGe signal, wherein the combined sEMGe signal represents a muscle potential activity of the subject.

7. The system as claimed in claim 6, wherein the one or more hardware processors are configured to preprocess the plurality of sEMGr signals to obtain the sEMG envelope by performing a DC offset removal, removal of any powerline noise and associated harmonics, band-pass filtering, full wave rectification, and low-pass filtering, of the sEMGr signals.

8. The system as claimed in claim 6, wherein the one or more hardware processors are configured to determine the onset and offset regions in the sEMGe signal from each of the plurality of channels, by performing the segmentation, comprises:

obtaining square waves associated with each of the plurality of channels, by applying an adaptive threshold-based segmentation algorithm on the sEMGe signal from each of the plurality of channels;
identifying time instances having a rising edge of the square wave as the onset of an active period; and
identifying time instances having a falling edge of the square wave as the offset of the active period.

9. The system as claimed in claim 8, wherein the one or more hardware processors are configured to generate the combined sEMGe signal by performing a logical OR operation of the square waves obtained for the plurality of channels.

10. The system as claimed in claim 6, wherein the one or more hardware processors are configured to decompose the sEMG envelope using a Variational Mode Decomposition (VMD) technique.

11. One or more non-transitory machine-readable information storage mediums comprising one or more instructions which when executed by one or more hardware processors cause:

receiving a plurality of raw sEMG signals (sEMGr) of a subject, as input;
preprocessing the plurality of sEMGr signals to obtain an sEMG envelope;
performing signal elevation on the sEMG envelope to obtain a conditioned signal, by:

decomposing the sEMG envelope into a plurality of Intrinsic Mode Functions (IMF); **characterized in that** the one or more instructions which when executed by one or more hardware processors cause
determining IMF having a) least value of noise, and b) a total power range with closest match with the sEMGr, from among the plurality of IMFs, as a candidate IMF; and
elevating the sEMG envelope by multiplying the sEMG envelope with the determined candidate IMF, to generate a plurality of sEMGe signals for a plurality of channels;

determining onset and offset regions in the sEMGe signal from each of the plurality of channels, by performing segmentation of the sEMGe signal from each of the plurality of channels; and
post-processing the sEMGe signals from the plurality of channels, to generate a combined sEMGe signal, wherein the combined sEMGe signal represents a muscle potential activity of the subject.

12. The one or more non-transitory machine-readable information storage mediums as claimed in claim 11, wherein preprocessing the plurality of sEMGr signals to obtain the sEMG envelope comprises of performing a DC offset removal, removal of any powerline noise and associated harmonics, band-pass filtering, full wave rectification, and low-pass filtering, of the sEMGr signals.

13. The one or more non-transitory machine-readable information storage mediums as claimed in claim 11, wherein determining the onset and offset regions in the sEMGe signal from each of the plurality of channels, by performing the segmentation, comprises:

obtaining square waves associated with each of the plurality of channels, by applying an adaptive threshold-based segmentation algorithm on the sEMGe signal from each of the plurality of channels;
identifying time instances having a rising edge of the square wave as the onset of an active period; and
identifying time instances having a falling edge of the square wave as the offset of the active period.

14. The one or more non-transitory machine-readable information storage mediums as claimed in claim 13, wherein generating the combined sEMGe signal comprises of performing a logical OR operation of the square waves obtained for the plurality of channels.

15. The one or more non-transitory machine-readable information storage mediums as claimed in claim 11, wherein the sEMG envelope is decomposed using a Variational Mode Decomposition (VMD) technique.

**Patentansprüche**

1. Prozessorimplementiertes Verfahren, umfassend:

Empfangen (202), über einen oder mehrere Hardwareprozessoren, einer Mehrzahl von rohen sEMG-Signalen (sEMGr) eines Subjekts als Eingabe;
Vorverarbeiten (204), über den einen oder die mehreren Hardwareprozessoren, der Mehrzahl von sEMGr-Signalen, um eine sEMG-Hüllkurve zu erhalten;
Durchführen (206), über den einen oder die mehreren Hardwareprozessoren, einer Signalerhöhung an der sEMG-Hüllkurve,
um ein konditioniertes Signal zu erhalten, durch:
Zerlegen (206a) der sEMG-Hüllkurve in eine Mehrzahl von intrinsischen Modusfunktionen (IMF); **dadurch gekennzeichnet, dass** das Verfahren ferner umfasst:

Bestimmen (206b) von IMF mit a) dem geringsten Rauschwert und b) einem Gesamtleistungsbereich mit der größten Übereinstimmung mit dem sEMGr aus der Mehrzahl von IMFs als Kandidaten-IMF; und
Erhöhen (206c) der sEMG-Hüllkurve durch Multiplizieren der sEMG-Hüllkurve mit dem bestimmten Kandidaten-IMF, um eine Mehrzahl von sEMGe-Signalen für eine Mehrzahl von Kanälen zu erzeugen;

Bestimmen (208), über den einen oder die mehreren Hardwareprozessoren, von Anfangs- und Versatzregionen in dem sEMGe-Signal von jedem der Mehrzahl von Kanälen durch Durchführen einer Segmentierung des sEMGe-Signals von jedem der Mehrzahl von Kanälen; und
Nachverarbeiten (210), über den einen oder die mehreren Hardwareprozessoren, der sEMGe-Signale von der Mehrzahl von Kanälen, um ein kombiniertes sEMGe-Signal zu erzeugen, wobei das kombinierte sEMGe-Signal eine Muskelpotentialaktivität des Subjekts darstellt.

2. Prozessorimplementiertes Verfahren nach Anspruch 1, wobei das Vorverarbeiten der Mehrzahl von sEMGr-Signalen, um die sEMG-Hüllkurve zu erhalten, ein Durchführen einer DC-Versatzentfernung, eine Entfernung von jeglichem Stromleitungsrauschen und zugehörigen Harmonien, eine Bandpassfilterung, eine Vollwellengleichrichtung und eine Tiefpassfilterung der sEMGr-Signale umfasst.

3. Prozessorimplementiertes Verfahren nach Anspruch 1, wobei das Bestimmen der Anfangs- und Versatzregionen in dem sEMGe-Signal von jedem der Mehrzahl von Kanälen durch Durchführen der Segmentierung umfasst:

Erhalten (302) von Rechteckwellen, die jedem der Mehrzahl von Kanälen zugeordnet sind, durch Anwenden eines adaptiven schwellenwertbasierten Segmentierungsalgorithmus auf das sEMGe-Signal von jedem der Mehrzahl von Kanälen;
Identifizieren (304) von Zeitinstanzen, die eine ansteigende Flanke der Rechteckwelle aufweisen, als den Anfang einer aktiven Periode; und
Identifizieren (306) von Zeitinstanzen, die eine abfallende Flanke der Rechteckwelle aufweisen, als den Versatz der aktiven Periode.

4. Prozessorimplementiertes Verfahren nach Anspruch 3, wobei das Erzeugen des kombinierten sEMGe-Signals ein Durchführen einer logischen ODER-Operation der Rechteckwellen, die für die Mehrzahl von Kanälen erhalten werden, umfasst.

5. Prozessorimplementiertes Verfahren nach Anspruch 1, wobei die sEMG-Hüllkurve unter Verwendung einer Variationsmoduszerlegungstechnik (VMD) zerlegt wird.

6. System (100), umfassend:

einen oder mehrere Hardwareprozessoren (102);
eine Kommunikationsschnittstelle (112); und
einen Speicher (104), der eine Mehrzahl von Anweisungen speichert,
wobei die Mehrzahl von Anweisungen den einen oder die mehreren Hardwareprozessoren zu Folgendem veranlassen:

Empfangen einer Mehrzahl von rohen sEMG-Signalen (sEMGr) eines Subjekts als Eingabe;
Vorverarbeiten der Mehrzahl von sEMGr-Signalen, um eine sEMG-Hüllkurve zu erhalten;
Durchführen einer Signalerhöhung an der sEMG-Hüllkurve, um ein konditioniertes Signal zu erhalten, durch:
Zerlegen der sEMG-Hüllkurve in eine Mehrzahl von intrinsischen Modusfunktionen (IMF) **dadurch gekennzeichnet, dass** die Mehrzahl von Anweisungen den einen oder die mehreren Hardwareprozessoren zu Folgendem veranlassen:

Bestimmen von IMF mit a) dem geringsten Rauschwert und b) einem Gesamtleistungsbereich mit der größten Übereinstimmung mit dem sEMGr aus der Mehrzahl von IMFs als Kandidaten-IMF; und
Erhöhen der sEMG-Hüllkurve durch Multiplizieren der sEMG-Hüllkurve mit dem bestimmten Kandidaten-IMF, um eine Mehrzahl von sEMGe-Signalen für eine Mehrzahl von Kanälen zu erzeugen;

Bestimmen von Anfangs- und Versatzregionen in dem sEMGe-Signal von jedem der Mehrzahl von Kanälen durch Durchführen einer Segmentierung des sEMGe-Signals von jedem der Mehrzahl von Kanälen; und
Nachverarbeiten der sEMGe-Signale von der Mehrzahl von Kanälen, um ein kombiniertes sEMGe-Signal zu erzeugen, wobei das kombinierte sEMGe-Signal eine Muskelpotentialaktivität des Subjekts darstellt.

7. System nach Anspruch 6, wobei der eine oder die mehreren Hardwareprozessoren konfiguriert sind, um die Mehrzahl von sEMGr-Signalen vorzuverarbeiten, um die sEMG-Hüllkurve zu erhalten, durch Durchführen einer DC-Versatzentfernung, einer Entfernung von jeglichem Stromleitungsrauschen und zugehörigen Harmonien, einer Bandpassfilterung, einer Vollwellengleichrichtung und einer Tiefpassfilterung der sEMGr-Signale.

8. System nach Anspruch 6, wobei der eine oder die mehreren Hardwareprozessoren konfiguriert sind, um die Anfangs- und Versatzregionen in dem sEMGe-Signal von jedem der Mehrzahl von Kanälen durch Durchführen der Segmentierung zu bestimmen, umfasst:

Erhalten von Rechteckwellen, die jedem der Mehrzahl von Kanälen zugeordnet sind, durch Anwenden eines adaptiven schwellenwertbasierten Segmentierungsalgorithmus auf das sEMGe-Signal von jedem der Mehrzahl von Kanälen;
Identifizieren von Zeitinstanzen, die eine ansteigende Flanke der Rechteckwelle aufweisen, als den Anfang einer aktiven Periode; und
Identifizieren von Zeitinstanzen, die eine abfallende Flanke der Rechteckwelle aufweisen, als den Versatz der aktiven Periode.

9. System nach Anspruch 8, wobei der eine oder die mehreren Hardwareprozessoren konfiguriert sind, um das kombinierte sEMGe-Signal durch Durchführen einer logischen ODER-Operation der Rechteckwellen, die für die Mehrzahl von Kanälen erhalten werden, zu erzeugen.

10. System nach Anspruch 6, wobei der eine oder die mehreren Hardwareprozessoren konfiguriert sind, um die sEMG-Hüllkurve unter Verwendung einer Variationsmoduszerlegungstechnik (VMD-Technik) zu zerlegen.

11. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes veranlassen:

Empfangen einer Mehrzahl von rohen sEMG-Signalen (sEMGr) eines Subjekts als Eingabe;
Vorverarbeiten der Mehrzahl von sEMGr-Signalen, um eine sEMG-Hüllkurve zu erhalten;
Durchführen einer Signalerhöhung an der sEMG-Hüllkurve, um ein konditioniertes Signal zu erhalten, durch:

Zerlegen der sEMG-Hüllkurve in eine Mehrzahl von intrinsischen Modusfunktionen (IMF); **dadurch gekennzeichnet, dass** die eine oder die mehreren Anweisungen, die bei Ausführung durch einen oder mehrere Hardwareprozessoren Folgendes veranlassen:

Bestimmen von IMF mit a) dem geringsten Rauschwert und b) einem Gesamtleistungsbereich mit der größten Übereinstimmung mit dem sEMGr aus der Mehrzahl von IMFs als Kandidaten-IMF; und Erhöhen der sEMG-Hüllkurve durch Multiplizieren der sEMG-Hüllkurve mit dem bestimmten Kandidaten-IMF, um eine Mehrzahl von sEMGe-Signalen für eine Mehrzahl von Kanälen zu erzeugen;

Bestimmen von Anfangs- und Versatzregionen in dem sEMGe-Signal von jedem der Mehrzahl von Kanälen durch Durchführen einer Segmentierung des sEMGe-Signals von jedem der Mehrzahl von Kanälen; und Nachverarbeiten der sEMGe-Signale von der Mehrzahl von Kanälen, um ein kombiniertes sEMGe-Signal zu erzeugen, wobei das kombinierte sEMGe-Signal eine Muskelpotentialaktivität des Subjekts darstellt.

12. Ein oder mehrere nichtflüchtige maschinenlesbare Informationsspeichermedien nach Anspruch 11, wobei das Vorverarbeiten der Mehrzahl von sEMGr-Signalen, um die sEMG-Hüllkurve zu erhalten, ein Durchführen einer DC-Versatzentfernung, eine Entfernung von jeglichem Stromleitungsrauschen und zugehörigen Harmonien, eine Bandpassfilterung, eine Vollwellengleichrichtung und eine Tiefpassfilterung der sEMGr-Signale umfasst.

13. Ein oder mehrere nichtflüchtige maschinenlesbare Informationsspeichermedien nach Anspruch 11, wobei das Bestimmen der Anfangs- und Versatzregionen in dem sEMGe-Signal von jedem der Mehrzahl von Kanälen durch Durchführen der Segmentierung umfasst:

Erhalten von Rechteckwellen, die jedem der Mehrzahl von Kanälen zugeordnet sind, durch Anwenden eines adaptiven schwellenwertbasierten Segmentierungsalgorithmus auf das sEMGe-Signal von jedem der Mehrzahl von Kanälen; Identifizieren von Zeitinstanzen, die eine ansteigende Flanke der Rechteckwelle aufweisen, als den Anfang einer aktiven Periode; und Identifizieren von Zeitinstanzen, die eine abfallende Flanke der Rechteckwelle aufweisen, als den Versatz der aktiven Periode.

14. Ein oder mehrere nichtflüchtige maschinenlesbare Informationsspeichermedien nach Anspruch 13, wobei das Erzeugen des kombinierten sEMGe-Signals ein Durchführen einer logischen ODER-Operation der Rechteckwellen, die für die Mehrzahl von Kanälen erhalten werden, umfasst.

15. Ein oder mehrere nichtflüchtige maschinenlesbare Informationsspeichermedien nach Anspruch 11, wobei die sEMG-Hüllkurve unter Verwendung einer Variationsmoduszerlegungstechnik (VMD-Technik) zerlegt wird.

**Revendications**

1. Procédé mis en oeuvre par processeur, comprenant :

la réception (202), via un ou plusieurs processeurs matériels, d'une pluralité de signaux sEMG bruts (sEMGr) d'un sujet, en tant qu'entrée ; le prétraitement (204), via les un ou plusieurs processeurs matériels, de la pluralité de signaux sEMGr pour obtenir une enveloppe sEMG ; l'exécution (206), via les un ou plusieurs processeurs matériels, d'une élévation de signal sur l'enveloppe sEMG pour obtenir un signal conditionné, par : la décomposition (206a) de l'enveloppe sEMG en une pluralité de fonctions de mode intrinsèque (IMF) ; **caractérisé en ce que** le procédé comprend en outre :

la détermination (206b) d'IMF ayant a) la plus faible valeur de bruit, et b) une plage de puissance totale avec la correspondance la plus proche avec le sEMGr, parmi la pluralité d'IMF, en tant qu'IMF candidate ; et l'élévation (206c) de l'enveloppe sEMG en multipliant l'enveloppe sEMG par l'IMF candidate déterminée, pour générer une pluralité de signaux sEMGe pour une pluralité de canaux ;

la détermination (208), via les un ou plusieurs processeurs matériels, de régions de début et de décalage dans le signal sEMGe à partir de chacun de la pluralité de canaux, en effectuant une segmentation du signal sEMGe à partir de chacun de la pluralité de canaux ; et

le post-traitement (210), via les un ou plusieurs processeurs matériels, des signaux sEMGe à partir de la pluralité de canaux, pour générer un signal sEMGe combiné, dans lequel le signal sEMGe combiné représente une activité potentielle musculaire du sujet.

2. Procédé mis en oeuvre par processeur selon la revendication 1, dans lequel le prétraitement de la pluralité de signaux sEMGr pour obtenir l'enveloppe sEMG comprend l'exécution d'une suppression de décalage DC, d'une suppression de tout bruit de ligne électrique et d'harmoniques associés, d'un filtrage passe-bande, d'un redressement pleine onde, et d'un filtrage passe-bas, des signaux sEMGr.

3. Procédé mis en oeuvre par processeur selon la revendication 1, dans lequel la détermination des régions de début et de décalage dans le signal sEMGe à partir de chacun de la pluralité de canaux, en effectuant la segmentation, comprend :

l'obtention (302) d'ondes carrées associées à chacun de la pluralité de canaux, en appliquant un algorithme de segmentation basé sur un seuil adaptatif sur le signal sEMGe à partir de chacun de la pluralité de canaux ;

l'identification (304) d'instances temporelles ayant un front montant de l'onde carrée en tant que début d'une période active ; et

l'identification (306) d'instances temporelles ayant un front descendant de l'onde carrée en tant que décalage de la période active.

4. Procédé mis en oeuvre par processeur selon la revendication 3, dans lequel la génération du signal sEMGe combiné comprend l'exécution d'une opération OR logique des ondes carrées obtenues pour la pluralité de canaux.

5. Procédé mis en oeuvre par processeur selon la revendication 1, dans lequel l'enveloppe sEMG est décomposée en utilisant une technique de décomposition en mode variationnel (VMD).

6. Système (100), comprenant :

un ou plusieurs processeurs matériels (102) ;

une interface de communication (112) ; et

une mémoire (104) stockant une pluralité d'instructions, dans lequel la pluralité d'instructions amènent les un ou plusieurs processeurs matériels à :

recevoir une pluralité de signaux sEMG bruts (sEMGr) d'un sujet, en tant qu'entrée ;

prétraiter la pluralité de signaux sEMGr pour obtenir une enveloppe sEMG ;

effectuer une élévation de signal sur l'enveloppe sEMG pour obtenir un signal conditionné, par :

décomposition de l'enveloppe sEMG en une pluralité de fonctions de mode intrinsèque (IMF) ; **caractérisé en ce que** la pluralité d'instructions amènent les un ou plusieurs processeurs matériels à :

déterminer l'IMF ayant a) la plus faible valeur de bruit, et b) une plage de puissance totale avec la correspondance la plus proche avec le sEMGr, parmi la pluralité d'IMF, en tant qu'IMF candidate ; et

élever l'enveloppe sEMG en multipliant l'enveloppe sEMG par l'IMF candidate déterminée, pour générer une pluralité de signaux sEMGe pour une pluralité de canaux ;

déterminer des régions de début et de décalage dans le signal sEMGe à partir de chacun de la pluralité de canaux, en effectuant une segmentation du signal sEMGe à partir de chacun de la pluralité de canaux ; et

post-traiter les signaux sEMGe à partir de la pluralité de canaux, pour générer un signal sEMGe combiné, dans lequel le signal sEMGe combiné représente une activité potentielle musculaire du sujet.

7. Système selon la revendication 6, dans lequel les un ou plusieurs processeurs matériels sont configurés pour prétraiter la pluralité de signaux sEMGr pour obtenir l'enveloppe sEMG en effectuant une suppression de décalage DC, une suppression de tout bruit de ligne électrique et d'harmoniques associés, un filtrage passe-bande, un redressement pleine onde, et un filtrage passe-bas, des signaux sEMGr.

8. Système selon la revendication 6, dans lequel les un ou plusieurs processeurs matériels sont configurés pour déterminer les régions de début et de décalage dans le signal sEMGe à partir de chacun de la pluralité de canaux, en effectuant la segmentation, comprend :

l'obtention d'ondes carrées associées à chacun de la pluralité de canaux, en appliquant un algorithme de segmentation basé sur un seuil adaptatif sur le signal sEMGe à partir de chacun de la pluralité de canaux ; l'identification d'instances temporelles ayant un front montant de l'onde carrée en tant que début d'une période active ; et l'identification d'instances temporelles ayant un front descendant de l'onde carrée en tant que décalage de la période active.

9. Système selon la revendication 8, dans lequel les un ou plusieurs processeurs matériels sont configurés pour générer le signal sEMGe combiné en effectuant une opération OR logique des ondes carrées obtenues pour la pluralité de canaux.

10. Système selon la revendication 6, dans lequel les un ou plusieurs processeurs matériels sont configurés pour décomposer l'enveloppe sEMG en utilisant une technique de décomposition en mode variationnel (VMD).

11. Un ou plusieurs supports de stockage d'informations lisibles par machine non transitoires comprenant une ou plusieurs instructions qui, lorsqu'elles sont exécutées par un ou plusieurs processeurs matériels, amènent :

la réception d'une pluralité de signaux sEMG bruts (sEMGr) d'un sujet, en tant qu'entrée ; le prétraitement de la pluralité de signaux sEMGr pour obtenir une enveloppe sEMG ; l'exécution d'une élévation de signal sur l'enveloppe sEMG pour obtenir un signal conditionné, par :

décomposition de l'enveloppe sEMG en une pluralité de fonctions de mode intrinsèque (IMF) ; **caractérisé en ce que** les une ou plusieurs instructions qui, lorsqu'elles sont exécutées par un ou plusieurs processeurs matériels, amènent IMF ayant a) la plus faible valeur de bruit, et b) une plage de puissance totale avec la correspondance la plus proche avec le sEMGr, parmi la pluralité d'IMF, en tant qu'IMF candidate ; et l'élévation de l'enveloppe sEMG en multipliant l'enveloppe sEMG par l'IMF candidate déterminée, pour générer une pluralité de signaux sEMGe pour une pluralité de canaux ;

la détermination de régions de début et de décalage dans le signal sEMGe à partir de chacun de la pluralité de canaux, en effectuant une segmentation du signal sEMGe à partir de chacun de la pluralité de canaux ; et le post-traitement des signaux sEMGe à partir de la pluralité de canaux, pour générer un signal sEMGe combiné, dans lequel le signal sEMGe combiné représente une activité potentielle musculaire du sujet.

12. Un ou plusieurs supports de stockage d'informations lisibles par machine non transitoires selon la revendication 11, dans lequel le prétraitement de la pluralité de signaux sEMGr pour obtenir l'enveloppe sEMG comprend l'exécution d'une suppression de décalage DC, d'une suppression de tout bruit de ligne électrique et d'harmoniques associés, d'un filtrage passe-bande, d'un redressement pleine onde, et d'un filtrage passe-bas, des signaux sEMGr.

13. Un ou plusieurs supports de stockage d'informations lisibles par machine non transitoires selon la revendication 11, dans lequel la détermination des régions de début et de décalage dans le signal sEMGe à partir de chacun de la pluralité de canaux, en effectuant la segmentation, comprend :

l'obtention d'ondes carrées associées à chacun de la pluralité de canaux, en appliquant un algorithme de segmentation basé sur un seuil adaptatif sur le signal sEMGe à partir de chacun de la pluralité de canaux ; l'identification d'instances temporelles ayant un front montant de l'onde carrée en tant que début d'une période active ; et l'identification d'instances temporelles ayant un front descendant de l'onde carrée en tant que décalage de la période active.

14. Un ou plusieurs supports de stockage d'informations lisibles par machine non transitoires selon la revendication 13, dans lequel la génération du signal sEMGe combiné comprend l'exécution d'une opération OR logique des ondes carrées obtenues pour la pluralité de canaux.

15. Un ou plusieurs supports de stockage d'informations lisibles par machine non transitoires selon la revendication 11, dans lequel l'enveloppe sEMG est décomposée en utilisant une technique de décomposition en mode variationnel

(VMD).

100

MEMORY 104

MODULES 106

REPOSITORY 110

108

I/O INTERFACE 112

HARDWARE PROCESSORS 102

FIG. 1A

FIG. 1B

202

receiving, via one or more hardware processors, a plurality of raw sEMG signals (sEMGr) of a subject, as input

204

preprocessing, via the one or more hardware processors, the plurality of sEMGr signals obtain an sEMG envelope

206

performing, via the one or more hardware processors, signal elevation on the sEMG envelope to obtain a conditioned signal, by:

206a

decomposing the sEMG envelope into a plurality of Intrinsic Mode Functions (IMF)

206b

determining IMF having a) least value of noise, and b) a total power range with closest match with the sEMGr, from among the plurality of IMFs, as candidate IMF

206c

elevating the sEMG envelope by multiplying the sEMG envelope with the determined candidate IMF, to generate a plurality of sEMGe signals for a plurality of channels

A

200

FIG. 2A

A

determining, via the one or more hardware processors, onset and offset regions in the sEMGe signal from each of the plurality of channels, by performing segmentation of the sEMGe signal from each of the plurality of channels

208

post-processing, via the one or more hardware processors, the sEMGe signals from the plurality of channels, to generate a combined sEMGe signal, wherein the combined sEMGe signal represents a muscle potential activity of the subject

210

200

FIG. 2B

302

obtaining square waves associated with each of the plurality of channels, by applying an adaptive threshold-based segmentation algorithm on the sEMGe signal from each of the plurality of channels

304

identifying time instances having a rising edge of the square wave as the onset of an active period

306

identifying time instances having a falling edge of the square wave as the offset of the active period

300

FIG. 3

FIG. 4A

FIG. 4B

FIG. 4C

FIG. 4D

FIG. 5A

FIG. 5B

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421006891

**Non-patent literature cited in the description**

- **YE Y et al.** Empirical mode decomposition: a method to reduce low frequency interferences from surface electroenterogram. *MEDICAL & BIOLOGICAL ENGINEERING & COMPUTING*, vol. 45 (6), 541-551